# EUROPÄISCHE PATENTSCHRIFT

(11) **EP 3 623 362 B1**
(45) Veröffentlichungstag und Bekanntmachung des Hinweises auf die Patenterteilung: **16.11.2022**
(21) Anmeldenummer: 19199519.0
(22) Anmeldetag: 12.12.2014
(51) Int. Cl.: C07D 231/12, C07B 41/08, C05G 3/90, C05C 1/02

(54) **NITRIFIKATIONSINHIBITOR ENTHALTENDE DÜNGEMITTELMISCHUNG**
NITRIFICATION INHIBITOR CONTAINING FERTILISER MIXTURE
INHIBITEUR DE NITRIFICATION CONTENANT UN MÉLANGE D'ENGRAIS

(30) Priorität: 13.12.2013 DE 102013020588
(43) Veröffentlichungstag der Anmeldung: 18.03.2020
(62) Teilanmeldung aus: 17161117.1
(73) Patentinhaber: EuroChem Agro GmbH, 68165 Mannheim (DE)
(72) Erfinder: Peters, Nils, 67227 Frankenthal (DE); Hähndel, Reinhardt, 67117 Limburgerhof (DE)
(74) Vertreter: Féaux de Lacroix, Stefan

(56) Entgegenhaltungen:
- WO-A1-2011/032904
- WO-A2-2013/121384
- DATABASE CA [Online] CHEMICAL ABSTRACTS SERVICE, COLUMBUS, OHIO, US; 1. Januar 1959 (1959-01-01), GRANDBERG, I. I. ET AL: "Pyrazoles. III. Addition of .alpha.,.beta.-unsaturated compounds to pyrazoles", XP002741584, gefunden im STN Database accession no. 1960:7187 & GRANDBERG, I. I. ET AL: "Pyrazoles. III. Addition of .alpha.,.beta.-unsaturated compounds to pyrazoles", ZHURNAL OBSHCHEI KHIMII , 29, 1099-104 CODEN: ZOKHA4; ISSN: 0044-460X, 1959,

## Beschreibung

Die Erfindung betrifft 2-(N-3,4-Dimethylpyrazol)bernsteinsäure (Isomerengemisch von 2-(3,4-Dimethyl-1H-pyrazol-1-yl)bernsteinsäure und 2-(2,3-Dimethyl-1H-pyrazol-1-yl)bernsteinsäure, im Verhältnis von vorzugsweise etwa 80 : 20, auch als DMPBS bezeichnet, oder eine der Einzelverbindungen) enthaltende Düngemittelmischungen auf Basis von Kalkammonsalpeter (KAS)-Mineraldüngemittel, sowie ein Verfahren zur Herstellung von DMPBS.

Um Pflanzen in der Landwirtschaft den von ihnen benötigten Stickstoff zur Verfügung zu stellen, werden häufig Ammoniumverbindungen enthaltende Düngemittel eingesetzt.

Ammoniumverbindungen werden im Boden in relativ kurzer Zeit mikrobiell zu Nitrat umgesetzt (Nitrifikation). Nitrat kann jedoch aus dem Boden ausgewaschen werden. Der ausgewaschene Anteil steht dabei für die Pflanzenernährung nicht mehr zur Verfügung, so dass aus diesem Grund eine schnelle Nitrifikation unerwünscht ist. Zur besseren Ausnutzung des Düngers werden deshalb dem Dünger Nitrifikationsinhibitoren zugesetzt. Eine bekannte Gruppe von Nitrifikationsinhibitoren sind Pyrazolverbindungen.

Ein Problem bei der Verwendung von Pyrazolverbindungen als Nitrifikationsinhibitoren ist deren hohe Flüchtigkeit. Bei der Lagerung von Pyrazolverbindungen enthaltenden Düngerzubereitungen tritt somit ein kontinuierlicher Verlust an Wirkstoff durch Verdampfung auf. Deshalb müssen die Pyrazolverbindungen durch geeignete Maßnahmen in einer nicht flüchtigen Form formuliert werden.

In der EP-B-1 120 388 sind Phosphorsäure-Additionssalze von 3,4-Dimethylpyrazol und 4-Chlor-3-methylpyrazol zur Verwendung als Nitrifikationsinhibitoren beschrieben. Durch die Salzform kann die Flüchtigkeit deutlich vermindert werden.

Die WO 96/24566 betrifft die Verwendung von schwerflüchtigen Pyrazolderivaten mit hydrophilen Gruppen als Nitrifikationsinhibitoren. Beispielsweise wird 2-(N-3-Methylpyrazol)bernsteinsäure als Nitrifikationsinhibitor vorgeschlagen. Als geeignete Mineraldünger werden ammoniumhaltige Nitrate, Sulfate oder Phosphate genannt. Die Toxizität dieses Nitrifikationsinhibitors erschwert seinen Einsatz, speziell bei höheren Einsatzkonzentrationen.

Die WO 2011/032904 und WO 2013/121384 beschreiben unter anderem DMPBS als Nitrifikationsinhibitoren.

Für KAS-Düngemittel geeignete Nitrifikationsinhibitoren sind bisher nicht bekannt, weshalb bislang KAS-Düngemittel ohne Nitrifikationsinhibitor eingesetzt werden.

Aufgabe der vorliegenden Erfindung ist die Bereitstellung einer KAS-Düngemittelmischung, die bei der Lagerung und Anwendung zu einem niedrigen Verlust an Nitrifikationsinhibitor führt. Ferner ist Aufgabe der vorliegenden Erfindung die Bereitstellung eines wirksamen Nitrifikationsinhibitors für KAS, der eine geringe Flüchtigkeit bei der Lagerung und Anwendung im Boden aufweist, sowie entsprechender KAS-Düngemittelmischungen. Ferner soll ein verbessertes Verfahren zur Herstellung von 2-(N-3,4-Dimethylpyrazol)bernsteinsäure bereitgestellt werden.

Die Aufgabe wird erfindungsgemäß gelöst durch eine Düngemittelmischung, enthaltend
A. Kalkammonsalpeter-Mineraldüngemittel, das, neben Ammoniumnitrat und Calciumcarbonat und/oder Magnesiumcarbonat und gegebenenfalls Wasser, bis zu 15 Gew.-%, bezogen auf das Kalkammonsalpeter-Mineraldüngemittel ohne Wasser, an weiteren Inhaltsstoffen aufweisen kann,
B. 100 bis 10000 Gew.-ppm, bezogen auf Komponente A ohne Wasser, an 2-(N-3,4-Dimethylpyrazol)bernsteinsäure.

Bevorzugt beträgt der Wasseranteil in Komponente A und in der Düngemittelmischung maximal 1,0 Gew.-%, besonders bevorzugt maximal 0,5 Gew.-%, insbesondere maximal 0,3 Gew.-% und ist somit in der Mengenbilanz vernachlässigbar. Die Komponenten A und B machen vorzugsweise mindestens 90 Gew.-%, besonders bevorzugt mindestens 95 Gew.-% der Düngemittelmischung aus.

Das Mineraldüngemittel kann neben Ammoniumnitrat Calciumcarbonat oder Magnesiumcarbonat oder ein Gemisch von Calciumcarbonat mit Magnesiumcarbonat enthalten.

Dabei und im folgenden Text beziehen sich Mengenangaben, insbesondere des Nitrifikationsinhibitors, vorzugsweise auf das feste Mineraldüngemittel A, auch wenn Wasser zusätzlich vorliegt, z. B. in Flüssig-Formulierungen.

Die Erfindung betrifft zudem ein Verfahren zur Herstellung einer derartigen Düngemittelmischung durch Einbringen von 2-(N-3,4-Dimethylpyrazol)bernsteinsäure in das Kalkammonsalpeter-Mineraldüngemittel und/oder Aufbringen von 2-(N-3,4-Dimethylpyrazol)bernsteinsäure auf das Kalkammonsalpeter-Mineraldüngemittel.

Die Erfindung betrifft ferner ein Verfahren zum Düngen von landwirtschaftlich oder gärtnerisch genutzten Erdböden, dadurch gekennzeichnet, dass man eine Düngemittelmischung, enthaltend
A. Kalkammonsalpeter-Mineraldüngemittel, das, neben Ammoniumnitrat und Calciumcarbonat und/oder Magnesiumcarbonat und gegebenenfalls Wasser, bis zu 15 Gew.-%, bezogen auf das Kalkammonsalpeter-Mineraldüngemittel ohne Wasser, an weiteren Inhaltsstoffen aufweisen kann,
B. 100 bis 10000 Gew.-ppm, bezogen auf Komponente A ohne Wasser, an 2-(N-3,4-Dimethylpyrazol)bernsteinsäure
oder die Komponenten A und B getrennt, aber innerhalb eines Zeitraums von 0 bis 5 Stunden, vorzugsweise 0 bis 1 Stunde, besonders bevorzugt etwa gleichzeitig, auf die Erdböden ausbringt.

Die Erfindung betrifft ferner ein Verfahren zur Herstellung von 2-(N-3,4-Dimethylpyrazol)bernsteinsäure durch Umsetzung von 3,4-Dimethylpyrazol mit Maleinsäure und/oder Maleinsäureanhydrid in Abwesenheit organischer Lösungs- oder Verdünnungsmittel und nachfolgende Kristallisation aus dem so erhaltenen Umsetzungsprodukt in Abwesenheit organischer Lösungs- oder Verdünnungsmittel.

Dabei können folgende Verfahren ausgeschlossen oder ausgenommen sein:
"Im ersten Versuch wurden 41,608 mol Maleinsäureanhydrid mit einer Reinheit von über 99,5 % vorgelegt und in 11 Litern destilliertem Wasser gelöst. Hierbei stieg die Temperatur um 10 °C an. Anschließend wurden 41,608 mol 80 %ige wässrige 3,4-Dimethylpyrazol-Lösung (laut NMR-Spektrum enthielt die eingesetzte Lösung des 3,4-DMPs ca. 2 % nicht näher charakterisierter Verunreinigungen) zugegeben, wobei die Temperatur um weitere 12 °C anstieg. Nach beendeter Zugabe wurde das Reaktionsgemisch auf 100 °C Innentemperatur erwärmt. Als diese Temperatur erreicht worden war, wurde das Reaktionsgemisch für 24 Stunden bei 100 °C gerührt und anschließend abkühlen gelassen. Nachdem das Reaktionsgemisch auf 90 °C abgekühlt war, wurde eine Probe für die NMR-spektroskopische Reaktionskontrolle entnommen und das Reaktionsgemisch anschließend mit 1 g Produkt (Kristalle von 2-(N-3,4-Dimethylpyrazol)bernstein-säure) angeimpft. Bei dieser Temperatur setzte noch keine Kristallisation ein, die zugegebenen Kristalle lösten sich aber auch nicht mehr auf.

Beim weiteren Abkühlen setzte ab circa 85 °C langsam die Kristallisation ein. Die Kristallisation der Hauptmenge des Produktes setzte erst knapp unterhalb von 80 °C unter Temperaturerhöhung ein. Das Reaktionsgemisch wurde zur vollständigen Kristallisation über Nacht unter Rühren abkühlen gelassen. Der ausgefallene Feststoff wurden über drei 8 L-G3-Glasfilternutschen mittels Saugflasche und Membranpumpe im Vakuum abfiltriert, mit insgesamt 8 Litern destilliertem Wasser gewaschen und anschließend bei 60 °C Badtemperatur im Vakuum getrocknet. Das so erhaltene trockene Produkt wurde in einen Behälter gegeben, gut durchmischt und eine Probe hiervon NMRspektroskopisch untersucht. In den nachfolgenden Versuchen wurde anstelle des destillierten Wassers eine entsprechende Menge der vereinigten Filtrate als Reaktionsmedium eingesetzt. Die überschüssige Menge der vereinigten Filtrate wurde entsorgt."

Diese ausgeschlossenen oder ausgenommenen Verfahren können aber auch eine erfindungsgemäße Alternative zum erfindungsgemäßen Verfahren mit Ausschluss/Ausnahme sein.

Die Erfindung betrifft zudem eine wässrige Lösung von 2-(N-3,4-Dimethylpyrazol)bernsteinsäure mit einem pH-Wert von größer 7.

2-(N-3,4-Dimethylpyrazol)bernsteinsäure ist vorzugsweise ein Isomerengemisch von 2-(3,4-Dimethyl-1H-pyrazol-1-yl)bernsteinsäure und 2-(2,3-Dimethyl-1H-pyrazol-1-yl)bernsteinsäure, vorzugsweise im Molverhältnis von 5 : 95 bis 95 : 5, besonders bevorzugt 50 : 50 bis 95 : 5, insbesondere 70 : 30 bis 90 : 10.

Es kann in der Säureform oder ganz oder teilweise neutralisiert bzw. ganz oder teilweise in Salzform vorliegen, z. B. als Alkalisalz, wie Kaliumsalz. Der erfindungsgemäß verwendete Begriff "2-(N-3,4-Dimethylpyrazol)bernsteinsäure" umfasst auch die teilweise oder vollständig(e) neutralisierte bzw. Salzform.

Es wurde erfindungsgemäß gefunden, dass die Kombination von 2-(N-3,4-Dimethylpyrazol)bernsteinsäure mit Kalkammonsalpeter-Mineraldüngemitteln zu einem wirksamen Nitrifikationsinhibitor führt, der bei der Lagerung und auch nach der Ausbringung auf den Boden eine verminderte Flüchtigkeit bzw. einen verminderten Verlust aufweist.

Ferner wurde 2-(N-3,4-Dimethylpyrazol)bernsteinsäure als besonders wirksamer Nitrifikationsinhibitor mit geringer Flüchtigkeit und geringer Toxizität aufgefunden. Daher betrifft die vorliegende Erfindung entsprechend die spezielle Kombination von 2-(N-3,4-Dimethylpyrazol)bernsteinsäure mit Kalkammonsalpeter-Düngemitteln.

Die Herstellung von 2-(N-3,4-Dimethylpyrazol)bernsteinsäure kann durch beliebige geeignete Verfahren erfolgen, die beispielsweise in allgemeiner Form in WO 96/24566 beschrieben sind. Vorzugsweise erfolgt die Herstellung durch Umsetzung von 3,4-Dimethylpyrazol mit Maleinsäure oder Maleinsäureanhydrid. Diese Umsetzung wird typischerweise in saurer Umgebung durchgeführt. Zur Herstellung von 3,4-Dimethylpyrazol kann auf Noyce et al., Jour. of Org. Chem. 20, 1955, Seiten 1681 bis 1682 verwiesen werden. Ferner kann auf EP-A-0 474 037, DE-A-3 840 342 und EP-A-0 467 707 verwiesen werden, wie auch auf EP-B-1 120 388.

Für die Reinigung des 3,4-Dimethylpyrazols kann auf DE-A-10 2009 060 150 verwiesen werden.

Günstigerweise nimmt man die Reaktion bei Temperaturen von 0 bis 150°C, vorzugsweise 50 bis 120°C, insbesondere 70 bis 105°C unter Normaldruck in Abwesenheit eines Lösungsmittels oder bevorzugt in einem inerten Lösungsmittel, wie Wasser, Acetonitril oder Dimethylsulfoxid vor. Weitere geeignete Lösungsmittel sind Alkohole, Ether, Ketone, Wasser sowie Alkane. Es kann sich auch die Umsetzung in einer organischen Säure wie Essigsäure anbieten. Das Produkt kann durch Umkristallisieren aufgereinigt werden, beispielsweise durch Aufnehmen durch Diethylether.

Es kann Maleinsäureanhydrid in Wasser gelöst und zur Maleinsäure umgesetzt werden. Dann kann eine wässrige Lösung von 3,4-Dimethylpyrazol zugesetzt werden. Die Umsetzung kann z. B. bei Temperaturen um 100°C erfolgen, z. B. bei 70 bis 105°C. Da 3,4-Dimethylpyrazol unter den Reaktionsbedingungen, in denen die Reaktion üblicherweise durchgeführt wird, tautomerisiert bzw. durch die Substitution am Stickstoff die 3,5-Tautomerie des Pyrazolrings aufgehoben ist, lässt es sich im Allgemeinen nicht vermeiden, Isomerengemische der erhaltenen substituierten Bernsteinsäure vorliegen zu haben, die Strukturisomere aufweisen.

Besonders bevorzugt erfolgt die Herstellung der 2-(N-3,4-Dimethylpyrazol)bernsteinsäure durch Umsetzung von 3,4-Dimethylpyrazol mit Maleinsäure, Maleinsäureanhydrid oder Maleinsäure/Maleinsäureanhydrid-Gemischen in Abwesenheit organischer Lösungs- oder Verdünnungsmittel und nachfolgende Kristallisation aus dem so erhaltenen Umsetzungsprodukt in Abwesenheit organischer Lösungs- oder Verdünnungsmittel. Sofern das Umsetzungsprodukt nach der Umsetzung nicht gelöst vorliegt, wird es vor der Kristallisation in einem nicht-organischen Lösungsmittel gelöst.

Es wurde erfindungsgemäß gefunden, dass das Produkt in hoher Ausbeute und Reinheit erhalten wird, wenn auf die Mitverwendung organischer Lösungs- oder Verdünnungsmittel bei der Herstellung und Kristallisation verzichtet wird.

Das Vorliegen geringer Mengen organischer Lösungs- oder Verdünnungsmittel bei der Umsetzung oder Kristallisation kann dabei toleriert werden. Erfindungsgemäß können bis zu 10 Gew.-%, besonders bevorzugt bis zu 5 Gew.-%, insbesondere bis zu 2,5 Gew.-% an organischen Lösungs- oder Verdünnungsmitteln, bezogen auf im Verfahren eingesetzte nicht-organische Lösungs- oder Verdünnungsmittel, toleriert werden. Besonders bevorzugt wird vollständig auf organische Lösungs- oder Verdünnungsmittel bei der Umsetzung und Kristallisation verzichtet. Hierdurch wird das Verfahren besonders umweltfreundlich.

Bevorzugt wird die Umsetzung in Wasser als Lösungsmittel durchgeführt, und die Kristallisation erfolgt aus dem (gelösten) wässrigen Umsetzungsprodukt.

Dabei können wässrige Lösungen oder Pasten von 3,4-Dimethylpyrazol und/oder Maleinsäure und/oder Maleinsäureanhydrid umgesetzt werden. Besonders bevorzugt werden sowohl 3,4-Dimethylpyrazol als auch Maleinsäure(anhydrid) als wässrige Lösungen oder Pasten eingesetzt. Einzelne Stoffe können auch als Feststoff eingesetzt werden. Beispielsweise kann 3,4-DMP auch als Feststoff eingesetzt werden.

Die Kristallisation erfolgt vorzugsweise durch Abkühlen des wässrigen Umsetzungsproduktes. Dabei können Impfkristalle mitverwendet werden, um die Kristallisation einzuleiten.

Die Umsetzung und Kristallisation können kontinuierlich oder diskontinuierlich durchgeführt werden. Es können ein oder mehrere Reaktoren bzw. Kristallisatoren eingesetzt werden. Beispielsweise kann eine Kaskade von Reaktoren und/oder Kristallisatoren eingesetzt werden. Eine chargenweise Umsetzung wie auch eine semikontinuierliche oder kontinuierliche Umsetzung und Kristallisation sind möglich.

Die nach der Kristallisation erhaltene 2-(N-3,4-Dimethylpyrazol)bernsteinsäure weist vorzugsweise eine Reinheit von mindestens 99,7%, besonders bevorzugt von mindestens 99,9% auf. Dabei wird diese Reinheit vorzugsweise bereits nach der ersten Kristallisation erreicht.

Durch die erfindungsgemäße Herstellung können eine große Ausbeute und eine hohe Reinheit mit geringem Aufwand erreicht werden. Insbesondere ist der Einsatz kostenintensiver und potentiell umweit- und gesundheitsschädlicher organischer Lösungs- und Verdünnungsmittel nicht notwendig. Auch ein Abtrennen oder Austausch von Lösungsmitteln ist nicht erforderlich.

Durch Verwendung des Umsetzungsprodukts von 3,4-Dimethylpyrazol mit Maleinsäure kann die Flüchtigkeit des 3,4-Dimethylpyrazols stark herabgesetzt werden.

Die Anwendung der 2-(N-3,4-Dimethylpyrazol)bernsteinsäure als Nitrifikationsinhibitor für KAS-Düngemittel erfolgt nach den allgemein üblichen Verfahren: Sie kann beispielsweise in fester Form in Kombination mit KAS-Düngemitteln als Pulver oder Granulat direkt auf den Boden aufgebracht werden. Sie kann zudem zu flüssigen KAS-Düngemitteln, z. B. in in Wasser gelöster Form, auch zur Stickstoffstabilisierung, zugesetzt werden oder gemeinsam mit ihnen in gelöster Form ausgebracht werden. Auch eine getrennte, aber zeitnahe Ausbringung von DMPBS und KAS-Düngemittel ist möglich.

Es hat sich besonders bewährt, Mischungen von 2-(N-3,4-Dimethylpyrazol)bernsteinsäure mit einem KAS-Mineraldüngemittel einzusetzen. Solche Düngemittelmischungen enthalten bevorzugt 100 bis 10000 Gew.-ppm, bezogen auf das Mineraldüngemittel, an Nitrifikationsinhibitor (0,01 bis 1 Gew.-%), besonders bevorzugt 0,03 bis 0,5 Gew.-%, insbesondere 0,05 bis 0,2 Gew.-%.

Die Düngemittelmischungen können auch geringe Mengen an Wasser enthalten, beispielsweise 0,1 bis 0,5 Gew.-%, bezogen auf die Düngemittelmischung inklusive Wasser. Hohe Wassermengen in der Düngemittelmischung sollten vermieden werden.

Besonders bewährt haben sich wegen ihrer guten Langzeitwirkung Düngemittelmischungen, die nach folgender Methode hergestellt werden:
Granulate von Mineraldüngemitteln, vorzugsweise Kalkammonsalpeter-Mineraldüngemittel, werden mit 2-(N-3,4-Dimethylpyrazol)bernsteinsäure imprägniert oder beschichtet, indem man sie mit einer Lösung des Nitrifikationsinhibitors besprüht und wieder trocknet. Die Methode ist beispielsweise aus DE-A-41 28 828 bekannt, auf die hier in vollem Umfang Bezug genommen wird. Die dort zusätzlich vorgeschlagene Versiegelung des imprägnierten Granulats mit einem Paraffinwachs erübrigt sich im Allgemeinen aufgrund der wesentlich geringeren Flüchtigkeit des erfindungsgemäßen Nitrifikationsinhibitors.

Die 2-(N-3,4-Dimethylpyrazol)bernsteinsäure kann auch bereits bei der Herstellung des Mineraldüngemittels zugesetzt werden, z. B. in der Maische.

Falls notwendig, kann auch eine Behandlung des Mineraldüngemittels mit Polysäuren erfolgen, wie sie in WO 98/05607/EP-B-0 971 526 beschrieben ist.

Üblicherweise werden die Nitrifikationsinhibitoren in Mengen von 100 g/ha bis 10 kg/ha auf den Boden aufgebracht.

Das Ausbringen in flüssigen Düngemittelformulierungen kann z. B. durch Fertigation mit oder ohne Überschusswasser erfolgen, wie in DE-C-102 30 593 beschrieben.

Das auf einfache Weise aus preisgünstigen Ausgangsprodukten herstellbare 2-(N-3,4-Dimethylpyrazol)bernsteinsäure zeichnet sich bei der Verwendung als Nitrifikationsinhibitor vor allem dadurch aus, dass es die Nitrifikation von Ammonium-Stickstoff im Boden über einen langen Zeitraum hinweg wirksam hemmt.

Hinzu kommt, dass diese Verbindung über günstige toxikologische Eigenschaften verfügt, einen niedrigen Dampfdruck aufweist und im Boden gut sorbiert wird. Dies hat zur Folge, dass 2-(N-3,4-Dimethylpyrazol)bernsteinsäure weder in nennenswertem Umfang durch Sublimation in die Atmosphäre abgegeben noch durch Wasser leicht ausgewaschen wird. Hierdurch ergeben sich zum einen ökonomische Vorteile wie eine hohe Wirtschaftlichkeit aufgrund der länger anhaltenden Wirkung des Nitrifikationsinhibitors und zum anderen ökologische Vorteile wie eine Verringerung der Belastung von Luft (Klimagas-reduzierend) und Oberflächengewässern und Grundwasser. Im Boden diffundiert 2-(N-3,4-Dimethylpyrazol)bernsteinsäure ähnlich schnell wie Nitrat bzw. Ammonium und kann daher optimal wirken. In der allgemeinsten Form können erfindungsgemäß beliebige geeignete Mineraldüngemittel eingesetzt werden. Dies sind Ammonium oder Harnstoff enthaltende Düngemittel. Beispiele derartiger Ammoniumhaltiger Düngemittel sind NPK-Düngemittel, Kalkammonsalpeter, Ammonsulfatsalpeter, Ammoniumsulfat oder Ammoniumphosphat.

Die nachfolgenden Mengenangaben beziehen sich auf das Mineraldüngemittel, bevorzugt ohne Wasser.

Besonders bevorzugt ist erfindungsgemäß eine Kombination von 2-(N-3,4-Dimethylpyrazol)bernsteinsäure mit Kalkammonsalpeter-Mineraldüngemittel. Dieses enthält Ammoniumnitrat und Kalziumcarbonat und/oder Magnesiumcarbonat als Hauptbestandteile und je nach Feuchtegrad Wasser. Es ist erfindungsgemäß möglich, dass das Kalkammonsalpeter-Mineraldüngemittel bis zu 15 Gew.-%, vorzugsweise bis zu 10 Gew.-%, besonders bevorzugt bis zu 5 Gew.-%, bezogen auf das Kalkammonsalpeter-Mineraldüngemittel ohne Wasser, an weiteren Inhaltsstoffen aufweisen kann. Weitere Inhaltsstoffe sind beispielsweise Spurenelemente, weitere Minerale, Stellmittel, Bindemittel usw.

Vorzugsweise beträgt der Gehalt an Stickstoff in Komponente A (ohne Wasser) 20 Gew.-%, bevorzugter mindestens 22 Gew.-%, besonders bevorzugt 25 bis 29 Gew.-%, insbesondere 26 bis 28 Gew.-%. Kalkammonsalpeter enthält häufig 26 bis 27 Gew.-% Stickstoff, wobei beispielsweise 13,5 Gew.-% schnell wirkender Nitratstickstoff und 13,5 Gew.-% langsam wirkender Ammoniumstickstoff vorliegen können.

Der Gehalt an Kalzium in Komponente A (ohne Wasser) beträgt beim Einsatz von Calciumcarbonat und Ammoniumnitrat als Inhaltsstoffen vorzugsweise 6 bis 15 Gew.-%, besonders bevorzugt 7 bis 13 Gew.-%, insbesondere 7 bis 11 Gew.-%. Typisch ist ein Gehalt von etwa 10 Gew.-%.

Beim Einsatz von Magnesium anstelle von Calcium im Carbonat kann eine entsprechende Menge an Mg bevorzugt vorliegen.

Gemäß einer bevorzugten Ausführungsform kann Komponente A bei Einsatz von Calciumcarbonat und Ammoniumnitrat als Inhaltsstoffen 0,5 bis 7 Gew.-%, vorzugsweise 1 bis 5 Gew.-%, besonders bevorzugt 3 bis 5 Gew.-%, bezogen auf Komponente A ohne Wasser, MgO und/oder Mg-Salz wie Magnesiumcarbonat enthalten. Typischerweise wird hier MgO oder MgCO₃ eingesetzt.

Ferner kann Komponente A gemäß einer Ausführungsform der Erfindung, bezogen auf Komponente A ohne Wasser, 0,1 bis 1 Gew.-%, vorzugsweise 0,1 bis 0,5 Gew.-%, insbesondere 0,15 bis 0,3 Gew.-%, Bor als Element und/oder in Form von Borverbindungen enthalten.

Für eine Beschreibung von Kalkammonsalpeter kann unter anderem auf die Definition in der EU-Düngemittel-Verordnung 2003/2003 verwiesen werden.

Bei Kalkammonsalpeter handelt es sich um einen weißen bis grauen Feststoff, der normalerweise geruchlos ist. Der pH-Wert einer 10%igen wässrigen Lösung liegt typischerweise bei mehr als 4,5. Der Schmelzpunkt liegt typischerweise im Bereich von 160 bis 170 °C je nach Feuchtigkeit. Die relative Dichte beträgt üblicherweise 0,93 bis 1,4 kg/l. Das Salz ist hygroskopisch und saugt Luftfeuchtigkeit auf.

Kalkammonsalpeter weist üblicherweise einen Wassergehalt von 0,1 bis 0,5 Gew.-%, bevorzugt 0,1 bis 0,2 Gew.-%, insbesondere etwa 0,15 Gew.-% auf. Durch das Aufbringen einer wässrigen Lösung von 2-(N-3,4-Dimethylpyrazol)bernsteinsäure auf das Kalkammonsalpeter-Mineraldüngemittel kann sich dieser Wassergehalt mehr als verdoppeln. Es kann dadurch notwendig sein, das so behandelte Kalkammonsalpeter-Mineraldüngemittel nach dem Aufbringen oder Einbringen des Nitrifikationsinhibitors zu trocknen.

Vorzugsweise wird die 2-(N-3,4-Dimethylpyrazol)bernsteinsäure als wässrige Lösung mit einem pH-Wert von größer 7, besonders bevorzugt größer 10, insbesondere größer 12 eingesetzt. Durch den basischen pH-Wert wird der Nitrifikationsinhibitor auf der Düngemittelmischung stabilisiert. Der pH-Wert kann beispielsweise durch Zufügen einer Base, insbesondere eines Alkalihydroxids, wie NaOH oder KOH, eingestellt werden.

Ferner wurde erfindungsgemäß gefunden, dass eine wässrige Lösung von 2-(N-3,4-Dimethylpyrazol)bernsteinsäure mit einem pH-Wert von größer 7, besonders bevorzugt größer 10, insbesondere größer 12, stabiler ist, so dass hochkonzentrierte wässrige Lösungen hergestellt werden können. Der Anteil an 2-(N-3,4-Dimethylpyrazol)bernsteinsäure, bezogen auf die wässrige Lösung, kann so vorzugsweise 20 bis 40 Gew.-%, besonders bevorzugt 25 bis 35 Gew.-%, insbesondere 27,5 bis 32,5 Gew.-% betragen.

Es wurde ferner erfindungsgemäß gefunden, dass durch Zusatz eines oder mehrerer Phosphate oder Polyphosphate zur wässrigen Lösung der Wasseranteil der wässrigen Lösung vermindert und die Stabilität der wässrigen Lösung des Nitrifikationsinhibitors nochmals verbessert werden kann. Vorzugsweise enthält daher die wässrige Lösung 0,5 bis 20 Gew.-%, besonders bevorzugt 1 bis 10 Gew.-%, insbesondere 1,5 bis 7 Gew.-%, bezogen auf die wässrige Lösung, eines oder mehrerer Phosphate oder Polyphosphate.

Als Phosphate kommen beispielsweise Na₂HPO₄, Na₃PO₄, K₂HPO₄, K₃PO₄, Diammoniumphosphat oder Kalziumammoniumphosphat in Betracht.

Die Erfindung betrifft auch die vorstehend beschriebenen wässrigen Lösungen von 2-(N-3,4-Dimethylpyrazol)bernsteinsäure mit einem pH-Wert von größer 7, sowie die bevorzugten Lösungen mit dem angegebenen Anteil an Nitrifikationsinhibitor und besonders bevorzugt Phosphaten oder Polyphosphaten.

Die Erfindung wird durch die nachstehenden Beispiele näher erläutert:

### Beispiele

### A. Herstellungsbeispiele

### Beispiel 1

9,6 g 3,4-Dimethylpyrazol (0,1 mol) und 9,8 g Maleinsäureanhydrid (0,1 mol) wurden in 50 ml 50%iger Essigsäure auf 100 °C erhitzt. Nach 16 h wurde zur Trockene eingedampft. Beim Aufnehmen des Rückstands in Diethylether fällt das Produkt (2-(N-3,4-Dimethylpyrazol)bernsteinsäure) rein aus und wird abfiltriert: Weiße Kristalle in einer Ausbeute von 92 %. Im NMR-Spektrum sind mehrere Methylsignale erkennbar, was mit der Aufhebung der 3,5-Tautomerie durch die Substitution an Stickstoff im Einklang steht.

### Beispiel 2: Herstellung im 200 kg-Maßstab

Als Ausgangsmaterialien für die Versuche wurden Maleinsäureanhydrid der CVM mit einer Reinheit von über 99,5 % und eine 80 %ige wässrige Lösung von 3,4-Dimethylpyrazol (3,4-DMP) der BASF SE eingesetzt. Laut NMR-Spektrum enthielt die eingesetzte Lösung des 3,4-DMPs ca. 2 % nicht näher charakterisierter Verunreinigungen.

Die Versuche wurden zunächst in einem 20 L-Reaktionsgefäß durchgeführt, das in weiteren Versuchen durch ein 25 L-Reaktionsgefäß ersetzt wurde.

Im ersten Versuch wurden 41,608 mol Maleinsäureanhydrid vorgelegt und in 11 Litern destilliertem Wasser gelöst. Hierbei stieg die Temperatur um 10 °C an. Anschließend wurden 41,608 mol 80 %ige wässrige 3,4-Dimethylpyrazol-Lösung zugegeben, wobei die Temperatur um weitere 12 °C anstieg. Nach beendeter Zugabe wurde das Reaktionsgemisch auf 100 °C Innentemperatur erwärmt. Als diese Temperatur erreicht worden war, wurde das Reaktionsgemisch für 24 Stunden bei 100 °C gerührt und anschließend abkühlen gelassen. Nachdem das Reaktionsgemisch auf 90 °C abgekühlt war, wurde eine Probe für die NMR-spektroskopische Reaktionskontrolle entnommen und das Reaktionsgemisch anschließend mit 1 g Produkt (Kristalle von 2-(N-3,4-Dimethylpyrazol)bernstein-säure) angeimpft. Bei dieser Temperatur setzte noch keine Kristallisation ein, die zugegebenen Kristalle lösten sich aber auch nicht mehr auf. Beim weiteren Abkühlen setzte ab circa 85 °C langsam die Kristallisation ein. Die Kristallisation der Hauptmenge des Produktes setzte erst knapp unterhalb von 80 °C unter Temperaturerhöhung ein. Das Reaktionsgemisch wurde zur vollständigen Kristallisation über Nacht unter Rühren abkühlen gelassen. Der ausgefallene Feststoff wurden über drei 8 L-G3-Glasfilternutschen mittels Saugflasche und Membranpumpe im Vakuum abfiltriert, mit insgesamt 8 Litern destilliertem Wasser gewaschen und anschließend bei 60 °C Badtemperatur im Vakuum getrocknet. Das so erhaltene trockene Produkt wurde in einen Behälter gegeben, gut durchmischt und eine Probe hiervon NMRspektroskopisch untersucht. In den nachfolgenden Versuchen wurde anstelle des destillierten Wassers eine entsprechende Menge der vereinigten Filtrate als Reaktionsmedium eingesetzt. Die überschüssige Menge der vereinigten Filtrate wurde entsorgt.

Die NMR-spektroskopische Reaktionskontrolle nach 24 Stunden ergab einen relativ konstanten Umsatz von circa 92 % mit einem relativ konstanten Isomeren-Verhältnis P1/P2 (2-(3,4-Dimethyl-1H-pyrazol-1-yl)bernsteinsäure/2-(2,3-Dimethyl-1H-pyrazol-1-yl)bernsteinsäure) um 3,3. Nur zu Beginn des Reihenversuchs lag das Verhältnis geringfügig höher. Dies war jedoch auch zu erwarten, da mit dem Einsatz des Filtrates anstelle des destillierten Wassers als Reaktionsmedium eine größere Menge an P2 (Verhältnis P1/P2 liegt in den Filtraten bei circa 1,0) in die nachfolgenden Versuche eingetragen wurde.

Die Zusammensetzung des Reaktionsgemisches nach 24 Stunden Reaktionszeit erreichte schon nach wenigen Versuchen konstante Werte. Ebenso weicht die Zusammensetzung der isolierten Produkte der einzelnen Versuche nur geringfügig voneinander ab.

Die im Mittel mit einer Ausbeute von 90,22 % erhaltenen Feststoffe besaßen eine Reinheit von 99,9 % und im Mittel ein Isomerenverhältnis von 4,0 (2-(3,4-Dimethyl-1H-pyrazol-1-yl)bernsteinsäure zu 2-(2,3-Dimethyl-1H-pyrazol-1-yl)bernsteinsäure). Verunreinigungen an 3,4-DMP, Maleinsäure und rac-Äpfelsäure waren in den 1H-NMR-Spektren nicht oder nur in Spuren (< 0,1 %) nachweisbar.

### Beispiel 3

Als Trägerdünger diente Kalkammonsalpeter mit 27 % N und 10 % Ca. 2 g 2-(N-3,4-Dimethylpyrazol)bernsteinsäure und 46 g KOH wurden in 133 g Wasser gelöst. 20 kg des Trägerdüngers in Form eines Granulats wurden in einer Trommel mit 85 g der Formulierung der Pyrazolverbindung langsam besprüht.

### Beispiel 4

Beispiel 3 wurde wiederholt, wobei anstelle von 133 g Wasser 111 g Wasser und 22 g Diammoniumphosphat eingesetzt wurden.

### Vergleichsbeispiel

Analog Beispiel 3 wurde 3,4-Dimethylpyrazolphosphat (DMPP) anstelle von 2-(N-3,4-Dimethylpyrazol)bernsteinsäure eingesetzt.

### B. Anwendungsbeispiele

### Beispiel 1

### Untersuchung der Lagerstabilität

Mit 2-(N-3,4-Dimethylpyrazol)bernsteinsäure (DMPBS) bzw. DMPP additiviertes Kalkammonsalpeter (KAS)-Mineraldüngemittel gemäß Beispiel 3 bzw. Vergleichsbeispiel wurde bezüglich der Lagerstabilität in einem Schnelltest untersucht, bei dem die nitrifikationsinhibierten Mineraldüngemittel in einem offenen Becherglas (das als Minihaufwerk die Lagersituation in einem großen Haufwerk nachstellt) für 40 Tage bei 30 °C, 40 bis 50 % relativer Luftfeuchtigkeit und etwa 1,2 m/s Luftgeschwindigkeit in einem gelüfteten Wärmeschrank gelagert wurden. Die Nitrifikationsinhibitorkonzentration auf dem Mineraldüngemittel wurde vor, während und nach der Lagerung in zwei unterschiedlichen Tiefen der Schüttung bestimmt und der Verlust an Nitrifikationsinhibitor ermittelt. Es wurden jeweils etwa 10 bis 30 g behandeltes Mineraldüngemittel gelagert. Die Konzentration an DMPP betrug dabei zu Beginn 1,028 g/kg Düngemittel, für 2-(N-3,4-Dimethylpyrazol)bernsteinsäure 1,244 g/kg Düngemittel.

Nach 20 und 40 Tagen wurden Proben aus einem oberflächlichen Bereich der Düngemittelschüttung (0 bis 5 cm Tiefe der Probenahme bzw. > 5 cm Tiefe der Probenahme) ermittelt.

Die Ergebnisse sind in der nachfolgenden Tabelle 1 dargestellt, wobei DMPBS 2-(N-3,4-Dimethylpyrazol)bernsteinsäure bedeutet.

**Tabelle 1**

| Lagerstabilität von DMPP und DMPBS auf KAS | |
|---|---|
| | Analysenwert [g/kg] |
| DMPP auf KAS | |
| Startwert | 1,028 |
| d20, 0 - 5 cm | 0,86 |
| d20, > - 5 cm | 0,91 |
| d40, 0 - 5 cm | 0,45 |
| d40, > - 5 cm | 0,68 |
| | |
| DMPBS auf KAS | |
| Startwert | 1,244 |
| d20, 0 - 5 cm | 1,15 |
| d20, > - 5 cm | 1,18 |
| d40, 0 - 5 cm | 1,21 |
| d40, > - 5 cm | 1,26 |

| | |
|---|---|
| d = Tag 0 - 5 cm Tiefe der Probenahme | |

Aus der Tabelle wird deutlich, dass der Verlust für 2-(N-3,4-Dimethylpyrazol)bernsteinsäure wesentlich niedriger ausfällt als für DMPP bei einer Lagerung über 20 bis 40 Tage.

Damit sind die Vorteile des erfindungsgemäßen Düngemittels belegt.

### Beispiel 2

Nachweis der biologischen (nitrifikationsinhibierenden) Wirkung der 2-(N-3,4-Dimethylpyrazol)bernsteinsäure

Die biologische Wirksamkeit von 2-(N-3,4-Dimethylpyrazol)bernsteinsäure wurde in mehreren Feldversuchen in unterschiedlichen Umwelten geprüft.

Für die Anlage, Beprobung, Beerntung und Auswertung der Feldversuche sind die im landwirtschaftlichen Versuchswesen üblichen Verfahren angewendet worden.

Die Analyse der Pflanzen- und Bodenproben erfolgte nach Standardmethoden. Die übrigen produktionstechnischen Maßnahmen, wie der Pflanzenschutz, entsprachen der guten landwirtschaftlichen Praxis und wurden einheitlich durchgeführt.

Ein biologisch wirksamer Nitrifikationsinhibitor zeichnet sich vorzugsweise dadurch aus, dass er in einem Zeitraum von bis zu 4 Wochen und länger nach der Applikation gegenüber der Kontrolle (hier nicht additivierter KAS-Trägerdünger) im Boden höhere Gehalte an NH₄-Stickstoff aufweist. Als Folge dieser Bedingungen ist der Ertrag erhöht und der Nitratgehalt der Pflanzen ist reduziert.

Es wurde der Wirkstoff analog Beispiel 3 auf feste KAS-Dünger mit einer Aufbringmenge von 0,73% bezogen auf den reduzierten Stickstoff aufgebracht. Der Wirkstoff zeigt eine starke nitrifikationshemmende Wirkung im Boden nach Ausbringung der Düngemittel. In dem in Tabelle 2 beispielhaft aufgeführten KAS (Kalkammonsalpeter) + DMPBS finden sich sowohl nach 14 Tagen als auch nach 28 Tagen im Vergleich zu unbeaufschlagten Produkten noch erhebliche Mengen an reduziertem Stickstoff, ohne Nitrifikationshemmstoff ist der gesamte reduzierte Stickstoff spätestens nach 28 Tagen nitrifiziert und zu Nitrat-N umgesetzt.

**Tabelle 2:**

| **Hemmung der Nitrifikation durch DMPBS** | | | |
|---|---|---|---|
| **Dünger** | % **NH₄-N (bzw. NH₂-N) vom gedüngten N nach** | | |
| | **0 Tage** | **14 Tagen** | **28 Tagen** |
| KAS | 100 | 9,1 | 0,0 |
| KAS + DMPBS | 100 | 79,3 | 61,9 |

### Beispiel 3

### Verminderung der Treibhausgasemissionen (N₂O)

Neben dem Schutz der Hydrosphäre ist auch eine möglichst weitgehende Vermeidung der Freisetzung von Klima-relevante Gasen infolge der landwirtschaftlichen Nutzung von Böden eine große Herausforderung für den Landbau.

Die Zusammenstellung der Messungen an Lachgas (N₂O), einem äußerst wirksamen Klimagases (ca. 300 mal stärker als CO₂), sowohl während der Vegetationsperiode von Winterweizen nach der Düngung als auch nach der Ernte bis in den Winter hinein, erbrachte im Vergleich zu herkömmlichem KAS bei der Verwendung von KAS+DMPBS gemäß Beispiel 3 eine Reduzierung um 28% (Tabelle 3).

**Tabelle 3:**

| **Wirkung einer Düngung mit KAS ohne und mit DMPBS auf die Freisetzung von Klimagas während einer Winterweizenkultur** | | |
|---|---|---|
| Ohne Düngung | KAS | KAS + DMPBS |
| g N₂O-N/ha kumuliert März bis Dezember | | |
| 1149 | 2690 | 1953 |
| 43% | 100% | 72% |

### Beispiel 4

### Wirkung auf Ertrag und Qualität von landwirtschaftlichen und gartenbaulichen Kulturen

### Erträge

Neben möglichen Auswirkungen auf die Schonung von Boden, Wasser und Luft ist für den Landwirt die Wirkung auf Ertrag und Qualität von besonderer Bedeutung. Die Zusammenstellung der gewogenen Erträge verschiedener Kulturen zeigt eine durchweg bessere Ertragsleistung der Dünger mit DMPBS gemäß Beispiel 3 als die Verwendung der jeweiligen herkömmlichen Dünger (Tabelle 4). Dabei gab es praktisch keine Unterschiede zwischen landwirtschaftlichen Kulturen und Gemüsekulturen oder von den jeweiligen Klimaräumen und verschiedenen Böden. Die Ursachen für die Mehrerträge liegen zum einen in den verminderten Verlusten durch Auswaschung und der gasförmigen Verluste durch Denitrifikation, zum anderen in der teilweisen Ammonium-Ernährung der Pflanzen, die sich vorteilhaft auf den pflanzlichen Stoffwechsel im Verleich zur üblichen Nitraternährung mit konventionellen Düngern auswirkt.

**Tabelle 4:**

| **Wirkung einer Düngung mit KAS ohne und mit DMPBS auf den Ertrag verschiedener gärtnerischer und landwirtschaftlicher Kulturen** | | | | | |
|---|---|---|---|---|---|
| **Kultur** | **Region/Land** | **Verwendeter Dünger** | **Ertrag dt/ha ohne** | **Ertrag dt/ha mit** | **Mehr-Ertrag [%]** |
| Kartoffel | Hannover/D | KAS | 464 | 609 | 31 |
| Kartoffel | Jütland/DK | KAS | 390 | 405 | 32 |
| Kartoffel | Picardie/F | KAS | 642 | 667 | 4 |
| Kartoffel | Orgiano/I | KAS | 531 | 582 | 9 |
| Kartoffel | Galicien/E | KAS | 644 | 728 | 13 |
| Sellerie* | Pfalz/D | KAS | 563 | 595 | 5 |
| Sellerie* | Pfalz/D | KAS | 756 | 781 | 3 |
| Chinakohl** | Pfalz/D | KAS | 757 | 842 | 11 |
| Chinakohl** | Pfalz/D | KAS | 817 | 930 | 13 |

| | | | | | |
|---|---|---|---|---|---|
| *Gewicht/100 Pfl. ** Gewicht pro Kopf, g | | | | | |

## Patentansprüche

1. Dialkalisalz der 2-(N-3,4-Dimethylpyrazol)bernsteinsäure.

2. Salz nach Anspruch 1, **dadurch gekennzeichnet, dass** das Dialkalisalz das Dikaliumsalz ist.

3. Düngemittelmischung, enthaltend
A. Kalkammonsalpeter-Mineraldüngemittel, das, neben Ammoniumnitrat und Calciumcarbonat und/oder Magnesiumcarbonat und gegebenenfalls Wasser, bis zu 15 Gew.-%, bezogen auf das Kalkammonsalpeter-Mineraldüngemittel ohne Wasser, an weiteren Inhaltsstoffen aufweisen kann,
B. 100 bis 10000 Gew.-ppm, bezogen auf Komponente A ohne Wasser, an Salz der 2-(N-3,4-Dimethylpyrazol)bernsteinsäure.

4. Düngemittelmischung nach Anspruch 3, **dadurch gekennzeichnet, dass** in Komponente A, bezogen auf das Kalkammonsalpeter-Mineraldüngemittel ohne Wasser, der Gehalt an Stickstoff mindestens 22 Gew.-%, vorzugsweise 25 bis 29 Gew.-%, besonders bevorzugt 26 bis 28 Gew.-% beträgt.

5. Düngemittelmischung nach einem der Ansprüche 3 oder 4, **dadurch gekennzeichnet, dass** in Komponente A, bezogen auf das Kalkammonsalpeter-Mineraldüngemittel ohne Wasser, der Gehalt an Kalzium und/oder Magnesium 6 bis 15 Gew.-%, vorzugsweise 7 bis 13 Gew.-%, insbesondere 9 bis 11 Gew.-%, beträgt.

6. Düngemittelmischung nach einem der Ansprüche 3 bis 5, **dadurch gekennzeichnet, dass** Komponente A 0,5 bis 7 Gew.-%, vorzugsweise 1 bis 5 Gew.-%, besonders bevorzugt 3 bis 5 Gew.-%, bezogen auf das Kalkammonsalpeter-Mineraldüngemittel ohne Wasser, MgO und/oder Mg-Salz enthält.

7. Düngemittelmischung nach einem der Ansprüche 3 bis 6, **dadurch gekennzeichnet, dass** Komponente A 0,01 bis 1 Gew.-%, vorzugsweise 0,1 bis 0,5 Gew.-%, insbesondere 0,15 bis 0,3 Gew-%, bezogen auf das Kalkammonsalpeter-Mineraldüngemittel ohne Wasser, Bor als Element oder in Form von Borverbindungen enthält.

8. Verfahren zur Herstellung einer Düngemittelmischung nach einem der Ansprüche 3 bis 7 durch Einbringen von 2-(N-3,4-Dimethylpyrazol)bernsteinsäure in Salzform in das Kalkammonsalpeter-Mineraldüngemittel und/oder Aufbringen von 2-(N-3,4-Dimethylpyrazol)bernsteinsäure in Salzform auf das Kalkammonsalpeter-Mineraldüngemittel.

9. Verfahren zum Düngen von landwirtschaftlich oder gärtnerisch genutzten Erdböden, **dadurch gekennzeichnet, dass** man eine Düngemittelmischung, enthaltend
A. Kalkammonsalpeter-Mineraldüngemittel, das, neben Ammoniumnitrat und Calciumcarbonat und/oder Magnesiumcarbonat und gegebenenfalls Wasser, bis zu 15 Gew.-%, bezogen auf das Kalkammonsalpeter-Mineraldüngemittel ohne Wasser, an weiteren Inhaltsstoffen aufweisen kann,
B. 100 bis 10000 Gew.-ppm, bezogen auf Komponente A ohne Wasser, an Salz der 2-(N-3,4-Dimethylpyrazol)bernsteinsäure
oder die Komponenten A und B getrennt, aber innerhalb eines Zeitraums von 0 bis 5 Stunden, auf die Erdböden ausbringt.

## Claims

1. Dialkali metal salt of 2-(N-3,4-dimethylpyrazole)succinic acid.

2. Salt according to claim 1, **characterized in that** the dialkali metal salt is the dipotassium salt.

3. Fertilizer mixture containing
A. Calcium ammonium nitrate mineral fertilizer which, in addition to ammonium nitrate and calcium carbonate and/or magnesium carbonate and optionally water, may comprise up to 15% by weight, based on the calcium ammonium nitrate mineral fertilizer without water, of further ingredients,
B. 100 to 10,000 ppm by weight, based on component A without water, of salt of 2-(N-3,4-dimethylpyrazole)succinic acid.

4. Fertilizer mixture according to claim 3, **characterized in that** the nitrogen content in component A is at least 22% by weight, preferably 25 to 29% by weight, particularly preferably 26 to 28% by weight, based on the calcium ammonium nitrate mineral fertilizer without water.

5. Fertilizer mixture according to one of claims 3 or 4, **characterized in that** the calcium and/or magnesium content in component A is 6 to 15% by weight, preferably 7 to 13% by weight, in particular 9 to 11% by weight, based on the calcium ammonium nitrate mineral fertilizer without water.

6. Fertilizer mixture according to one of claims 3 to 5, **characterized in that** component A contains 0.5 to 7% by weight, preferably 1 to 5% by weight, particularly preferably 3 to 5% by weight, based on the calcium ammonium nitrate mineral fertilizer without water, of MgO and/or Mg salt.

7. Fertilizer mixture according to one of claims 3 to 6, **characterized in that** component A contains 0.01 to 1% by weight, preferably 0.1 to 0.5% by weight, in particular 0.15 to 0.3% by weight, based on the calcium ammonium nitrate mineral fertilizer without water, of boron as element or in the form of boron compounds.

8. Process for preparing a fertilizer mixture according to any one of claims 3 to 7 by introducing 2-(N-3,4-dimethylpyrazole)succinic acid in salt form into the calcium ammonium nitrate mineral fertilizer and/or applying 2-(N-3,4-dimethylpyrazole)succinic acid in salt form to the calcium ammonium nitrate mineral fertilizer.

9. Process for fertilizing soil used for agriculture or horticulture, **characterized in that** a fertilizer mixture comprising
A. Calcium ammonium nitrate mineral fertilizer which, in addition to ammonium nitrate and calcium carbonate and/or magnesium carbonate and optionally water, may comprise up to 15% by weight, based on the calcium ammonium nitrate mineral fertilizer without water, of further ingredients,
B. 100 to 10,000 ppm by weight, based on component A without water, of salt of 2-(N-3,4-dimethylpyrazole)succinic acid
or the components A and B separately, but within a period of 0 to 5 hours, is deployed on the soil.

## Revendications

1. Sel de di(métal alcalin) d'acide 2-(N-3,4-diméthylpyrazole)succinique.

2. Sel selon la revendication 1, **caractérisé en ce que** le sel de di(métal alcalin) est le sel de dipotassium.

3. Mélange d'engrais, contenant
A. un engrais minéral nitrate d'ammonium calcique qui, en plus de nitrate d'ammonium et de carbonate de calcium et/ou carbonate de magnésium et éventuellement d'eau, peut comporter jusqu'à 15 % en poids, par rapport à l'engrais minéral nitrate d'ammonium calcique sans eau, d'autres composants,
B. 100 à 10 000 ppm en poids, par rapport au composant A sans eau, de sel de l'acide 2-(N-3,4-diméthylpyrazole)succinique.

4. Mélange d'engrais selon la revendication 3, **caractérisé en ce que**, dans le composant A, par rapport à l'engrais minéral nitrate d'ammonium calcique sans eau, la teneur en azote est d'au moins 22 % en poids, de préférence de 25 à 29 % en poids, de façon particulièrement préférée de 26 à 28 % en poids.

5. Mélange d'engrais selon l'une quelconque des revendications 3 et 4, **caractérisé en ce que**, dans le composant A, par rapport à l'engrais minéral nitrate d'ammonium calcique sans eau, la teneur en calcium et/ou magnésium vaut de 6 à 15 % en poids, de préférence de 7 à 13 % en poids, en particulier de 9 à 11 % en poids.

6. Mélange d'engrais selon l'une quelconque des revendications 3 à 5, **caractérisé en ce que** le composant A contient 0,5 à 7 % en poids, de préférence 1 à 5 % en poids, de façon particulièrement préférée 3 à 5 % en poids, par rapport à l'engrais minéral nitrate d'ammonium calcique sans eau, de MgO et/ou d'un sel de Mg.

7. Mélange d'engrais selon l'une quelconque des revendications 3 à 6, **caractérisé en ce que** le composant A contient 0,01 à 1 % en poids, de préférence 0,1 à 0,5 % en poids, en particulier 0,15 à 0,3 % en poids, par rapport à l'engrais minéral nitrate d'ammonium calcique sans eau, de bore sous forme élémentaire ou sous la forme de composés de bore.

8. Procédé pour la production d'un mélange d'engrais selon l'une quelconque des revendications 3 à 7 par introduction d'acide 2-(N-3,4-diméthylpyrazole)-succinique sous forme de sel dans l'engrais minéral nitrate d'ammonium calcique et/ou application d'acide 2-(N-3,4-diméthylpyrazole)succinique sous forme de sel sur l'engrais minéral nitrate d'ammonium calcique.

9. Procédé pour la fertilisation de sols exploités en agriculture ou horticulture, **caractérisé en ce qu'**on applique sur les sols un mélange d'engrais contenant
A. un engrais minéral nitrate d'ammonium calcique qui, en plus de nitrate d'ammonium et de carbonate de calcium et/ou carbonate de magnésium et éventuellement d'eau, peut comporter jusqu'à 15 % en poids, par rapport à l'engrais minéral nitrate d'ammonium calcique sans eau, d'autres composants,
B. 100 à 10 000 ppm en poids, par rapport au composant A sans eau, de sel de l'acide 2-(N-3,4-diméthylpyrazole)succinique
ou les composants A et B séparément, mais en un espace de temps de 0 à 5 heures,
